(19) Europäisches Patentamt
European Patent Office
Office européen des brevets

(11) Numéro de publication: **0 190 524 B1**

(12) # FASCICULE DE BREVET EUROPEEN

(45) Date de publication de fascicule du brevet: **06.03.91**

(51) Int. Cl.[5]: **C07D 403/00**, C07D 207/00, C07C 233/00

(21) Numéro de dépôt: **85402512.9**

(22) Date de dépôt: **17.12.85**

(54) **Nouveau procédé industriel de synthèse du N-[(1'-allyl 2'-pyrrolidinyl) méthyl] 2-méthoxy 4,5-azimido benzamide.**

(30) Priorité: **18.12.84 FR 8419322**

(43) Date de publication de la demande: **13.08.86 Bulletin 86/33**

(45) Mention de la délivrance du brevet: **06.03.91 Bulletin 91/10**

(84) Etats contractants désignés: **AT BE CH DE FR GB IT LI LU NL SE**

(56) Documents cités:
**FR-A- 2 097 041**
**GB-A- 2 083 459**
**US-A- 3 839 330**

(73) Titulaire: **SOCIETE D'ETUDES SCIENTIFIQUES ET INDUSTRIELLES DE L'ILE-DE-FRANCE**
**46, Boulevard de Latour-Maubourg**
**F-75340 Paris Cédex 07(FR)**

(72) Inventeur: **Acher, Jacques**
**32, route de Saint-Vrain**
**F-91760 Itteville(FR)**
Inventeur: **Monier, Jean-Claude**
**6, rue de la Sorbonne**
**F-91510 Lardy(FR)**

EP 0 190 524 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne un nouveau procédé de synthèse du N -[ (1'-allyl 2'-pyrrolidinyl) méthyl] 2-méthoxy 4,5-azimidobenzamide, de formule :

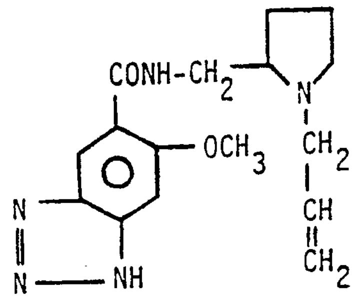

Ce composé, décrit pour la première fois dans le brevet français Fr-A-2 297 041, est utile comme médicament, notamment dans le domaine gastro-entérologique.

D'après le brevet US-A-3839330, on connaissait un procédé de préparation de dérivés de 4,5-azimido benzamide consistant à préparer un 4-amino 5-nitro benzamide puis à former le cycle azimido en plusieurs étapes.

La réaction d'amidification était l'une des premières étapes de ce procédé.

Des techniques plus adaptées à la préparation du N-[ (1'-allyl 2'-pyrrolidinylméthyl) ] 2-méthoxy 4,5-azimido benzamide ont donc été recherchées et sont décrites ou mentionnées dans le brevet Fr-A-2297041.

Selon ce brevet, le N-[(1'allyl 2'-pyrrolidinyl méthyl) ] 2-méthoxy 4,5-azimido benzamide est préparé par des méthodes dont la dernière étape consiste à amidifier l'acide 2-méthoxy 4,5-azimido benzoïque par la 1-allyl-2-aminométhyl pyrrolidine, soit directement soit en passant par l'intermédiaire d'un dérivé réactif de cet acide, ou de cette amine.

Par exemple, on a décrit la réaction de l'acide 2-méthoxy 4,5-azimido benzoïque avec des produits obtenus par réaction préalable de l'amine sur des chlorures de phosphore, sur l'oxychlorure de phosphore, sur des chlorophosphites substitués, ou encore par réaction de l'acide avec l'isocyanate de l'amine. De la même façon, il a été utilisé des esters (d'alkyle, ou cyanométhylique, ou méthoxyméthylique) ou encore des imides, des azides, des anhydrides d'acide mixtes ou symétriques, et d'autres dérivés de l'acide 2-méthoxy 4,5-azimido benzoïque réagissant sur la N-allyl 2-aminométhyl pyrrolidine elle-même. Or tous ces procédés nécessitent la mise en oeuvre d'une matière première peu répandue sur le marché, et dont l'éventuelle pénurie serait très préjudiciable à la synthèse du composé souhaité. Afin de prévenir un tel incident, ou toute autre difficulté inhérente à un problème de fabrication, il a été mis au point une méthode alternative de synthèse, applicable industriellement, pour assurer une production convenable du N-[ (1'-allyl 2'-pyrrolidinyl) méthyl ] 2-méthoxy 4,5-azimido benzamide.

D'après le brevet GB-A-2083459, on connaissait une méthode de préparation de dérivés de N-(2,5-dihalopentyl) benzamide par réaction d'un dérivé réactif de l'acide benzoïque substitué correspondant avec une dihalopentylamine et l'utilisation des composés ainsi obtenus pour préparer des dérivés de N-(2-pyrrolidinylméthyl) benzamide par réaction avec l'amine appropriée.

Ce brevet comprend, dans la formule générale des composés qui peuvent être préparés selon le procédé décrit, le N-(2,5-dichloropentyl) 2-méthoxy 4,5-diacétylaminobenzamide et le N-(2,5-dichloropentyl) 2-méthoxy 4,5-azimido benzamide. Cependant, le procédé de préparation décrit consistant à faire réagir avec la 2,5-dichloropentylamine, le dérivé d'acide benzoïque déjà convenablement substitué, ce dernier ne comprend pas la préparation d'un 4,5-azimido benzamide à partir d'un 4,5-diacétylamino benzamide.

Le procédé de l'invention présente un autre avantage important par rapport aux méthodes connues.

En effet, dans celles-ci il existe une limite de production liée à l'obligation d'utiliser un autoclave pour réduire l'intermédiaire 5-nitré en intermédiaire 5-aminé. La présente méthode élimine cet inconvénient en ne nécessitant pas de travailler sous pression.

Elle permet donc de fabriquer de grandes quantités de produit en une seule opération, ce qui constitue un avantage industriel important puisque l'on n'est plus limité par la taille d'un réacteur spécifique. C'est cette méthode qui fait l'objet de l'invention.

On peut la résumer par le schéma réactionnel suivant :

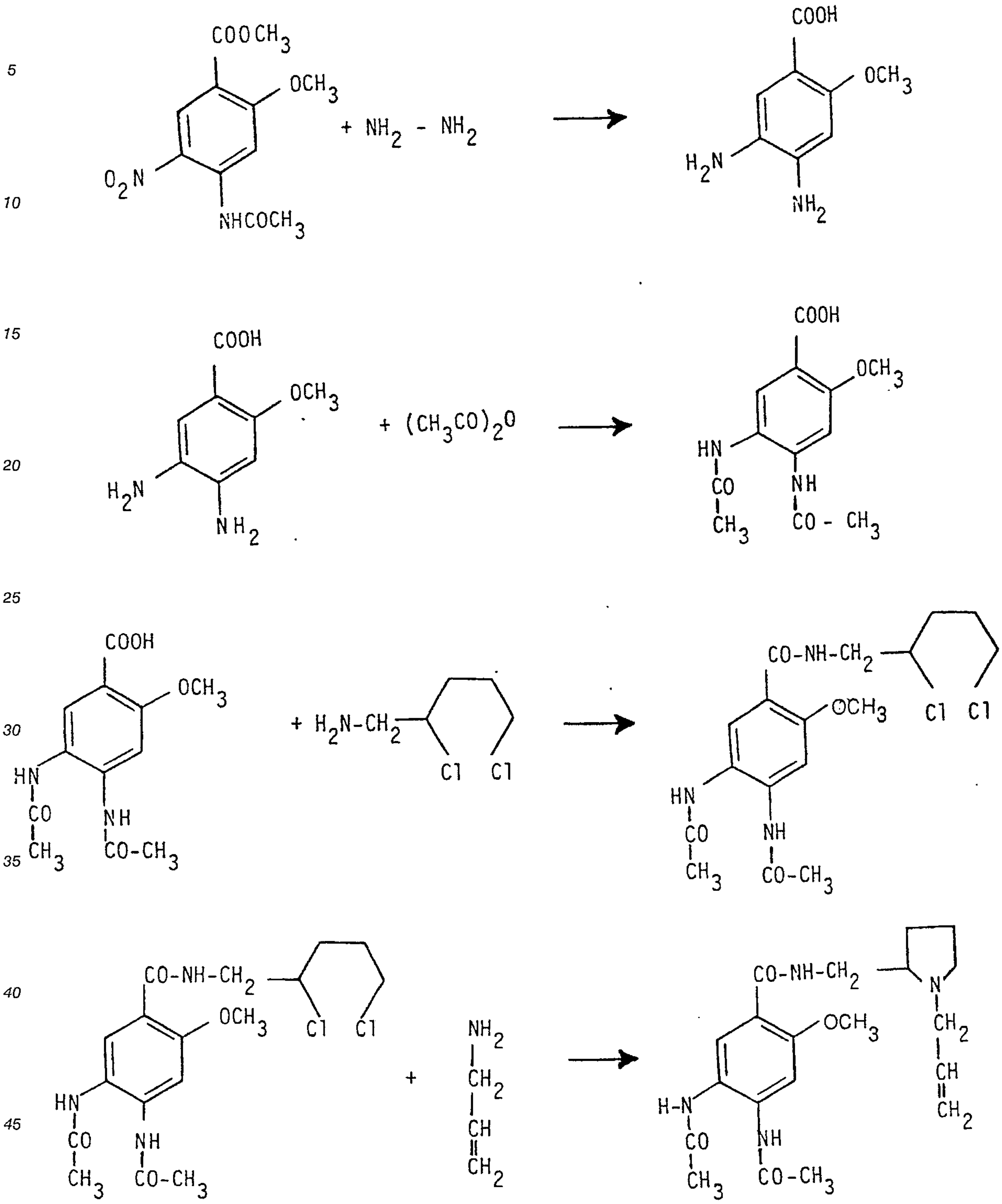

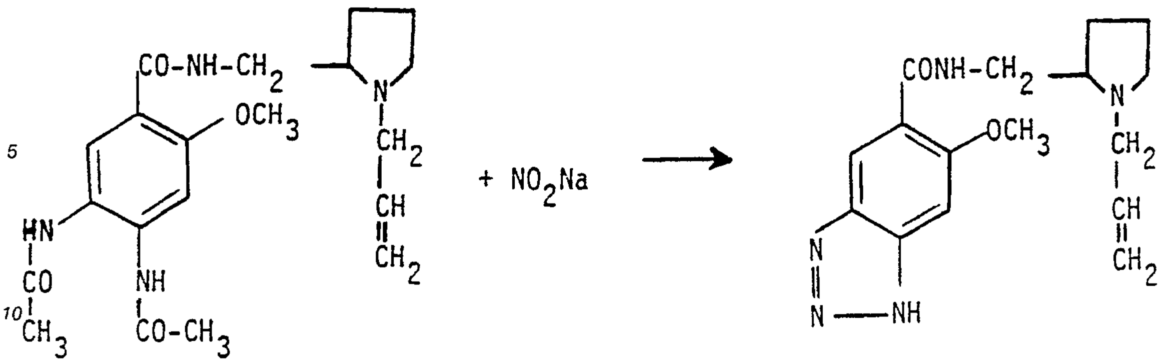

Le procédé consiste à faire réagir le 2-méthoxy 4-acétylamino 5-nitrobenzoate de méthyle avec de la soude pour obtenir le 2-méthoxy 4-amino 5-nitrobenzoate de sodium, qu'on traite par de l'hydrate d'hydrazine en présente de Nickel de Raney. L'acide 2-méthoxy 4,5-diamino benzoïque obtenu est 4,5 diacétylé au moyen d'anhydride acétique : la fonction acide est activée sous forme d'anhydride mixte par le chloroformiate d'éthyle, et cet anhydride est traité par la dichloropentylamine pour donner le N (2', 5'-dichloropentyl) 2-méthoxy 4,5-diacétylamino benzamide. Celui-ci, par réaction avec l'allylamine, donne le N - [ (1'-allyl 2'-pyrrolidinyl) méthyl] 2-méthoxy 4,5-diacétylamino benzamide qu'on transforme en N - [(1'-allyl 2'-pyrrolidinyl) méthyl] 2-méthoxy 4,5-azimido benzamide par traitement au nitrite de sodium.

La matière première 2-méthoxy 4-acétylamino 5-nitrobenzoate de méthyle est connue en soi.

L'acide 2-méthoxy 4,5-diacétylamino benzoïque, le N - (2',5'-dichloropentyl) 2-méthoxy 4,5-diacétylamino benzamide, et le N - [(1'allyl 2'-pyrrolidinyl) méthyl] 2-méthoxy 4,5-diacétylamino benzamide, sont des composés nouveaux.

Les exemples ci-après illustrent l'invention.

Acide 2-méthoxy 4,5-diamino benzoïque

Dans un ballon de quatre litres muni d'un agitateur, d'un thermomètre, d'un réfrigérant et d'une ampoule à brome, on a introduit 132,3 g (0,494 mole) de 2-méthoxy 4-acétylamino 5-nitrobenzoate de méthyle, 1320 ml d'eau, et 130 g de lessive de soude à 40 %.

On a chauffé quatre heures à reflux (température dans la masse 100° C), laissé redescendre vers 90° C, ajouté 12,5 g de Nickel Raney et coulé goutte à goutte 51,6 g (1,03 mole) d'hydrate d'hydrazine de façon à maintenir la température de réaction vers 90° C sans apport extérieur de chaleur. (La solution doit être totalement décolorée à la fin de l'addition, sinon, ajouter encore un peu d'hydrazine pour permettre cette décoloration). On a laissé ensuite une heure supplémentaire à reflux, et reposer une nuit. On a ajouté du noir de carbone végétal, filtré, lavé le noir trois fois avec 40 ml d'eau et acidifié le filtrat à pH = 4,9 avec 9,7 g (8,2 ml) d'acide chlorhydrique d = 1,18. On a laissé cristalliser le produit trois heures au réfrigérateur, essoré, lavé les cristaux quatre fois avec 120 ml d'eau et on les a séchés une nuit à l'étuve à 50° C.

Rendement = 79 à 82 g (88 % - 91 %)
P F = 198° C

Acide 2-méthoxy 4,5-diacétylamino benzoïque

Dans un ballon de deux litres, muni d'un agitateur, d'un thermomètre, d'un réfrigérant et d'une ampoule à brome, on a introduit 142 g (0,78 mole) d'acide 2-méthoxy 4,5-diamino benzoïque et 710 ml d'eau. On a porté la suspension à 45-50° C et ajouté goutte à goutte 442 g (4,33 moles) d'anhydride acétique. La suspension s'est décolorée et a épaissi. On a laissé ensuite agiter 1,5 heure à 50 ± 1° C, et reposer une nuit. On a essoré le solide qui a été lavé à l'eau et séché à l'étuve à 50° C.

Rendement = 174-178 g. (84 % - 86 %)
P F > 260° C (Kofler)

Le spectre RMN était compatible avec la structure du produit attendu.

N - (2',5'-dichloropentyl) 2-méthoxy 4,5-diacétylamino benzamide

Dans un ballon de 500 ml muni d'un agitateur, d'un thermomètre, d'un réfrigérant et d'une ampoule à brome, on a introduit 26,6 g (0,1 mole) d'acide 2-méthoxy 4,5-diacétylamino benzoïque, 260 ml d'acétone, 13,9 ml de triéthylamine et 60 ml (56,7 g) de diméthylformamide. On a agité à température ambiante pendant 2,5 heures (jusqu'à dissolution totale de l'acide), refroidi à 0° C et coulé goutte à goutte 10,85 g (9,5 ml) soit 0,1 mole de chloroformiate d'éthyle. On a laissé réagir 45 mn à 0° C, ajouté 21,3 g (0,110 mole) de chlorhydrate de 2,5 dichloro pentylamine par portions en 5 minutes puis goutte à goutte 15,3 ml de triéthylamine (soit en tout 29,2 ml ou 21,2 g = 0,210 mole). On a laissé revenir à température ambiante, agité trois heures et versé la suspension dans 550 ml d'eau. Il y a eu solubilisation suivie immédiatement de l'apparition d'un précipite. On a laissé cristalliser une nuit au réfrigérateur et essoré le précipité qui a été lavé 5 fois avec 60 ml d'eau puis séché à l'étuve à 50° C.

Rendement = 33 g. (82 %)
P F = 154-157° C

Le spectre RMN était compatible avec la structure du produit attendu.

C1 % - Trouvé = 17,48 - Calculé = 17,57

N - [(1'-allyl 2'-pyrrolidinyl) méthyl 2-méthoxy 4,5-diacétylamino benzamide

Dans un ballon de 250 ml muni d'un agitateur, d'un thermomètre et d'un réfrigérant, on a introduit 16,2 g (0,04 mole) de N (2',5'dichloropentyl) 2-méthoxy 4,5-diacétylamino benzamide et 46 g (60 ml = 0,8 mole) d'allylamine.

On a laissé agiter la suspension à 20° C puis trois heures à reflux. L'allylamine en excès a été évaporée et l'huile résiduelle a été reprise dans 100 ml d'eau à 40° C. Le produit a cristallisé. On a ajouté 8 ml de lessive de soude à 40 % et on a laissé cristalliser trois heures à 20° C. On a essoré, lavé à l'eau et séché le solide à l'étuve à 50° C.

Rendement = 11,2 g. (72 %)

Ce produit a été recristallisé dans un mélange de 200 ml d'acétate d'éthyle et 20 ml d'éthanol, à l'ébullition (PF = 155° C). Le spectre RMN était compatible avec la structure du produit attendu.

N - [(1'-allyl 2'-pyrrolidinyl) méthyl ] 2-méthoxy 4,5-azimido benzamide

Dans un ballon de 500 ml muni d'un agitateur, d'un thermomètre et d'une ampoule à brome, on a introduit 112 ml de méthanol et 3,10 g (0,045 mole) de nitrite de sodium. On a agité pour dissoudre puis ajouté une solution de 11,6g (0,03 mole) de N - [(1'allyl 2'-pyrrolidinyl) méthyl ] - 2-méthoxy 4,5-diacétylamino benzamide dans 75 ml de méthanol.

On a ensuite ajouté lentement, en refroidissant pour maintenir la température à 20° C, 63,6 ml (0,135 mole) de méthanol chlorhydrique 2,12 N. La solution limpide au début s'est troublée et, après 48 heures de repos à température ambiante, on a évaporé le solvant sous vide. Le résidu huileux a été dissous à chaud dans 50 ml d'isopropanol, 2,7 g de chlorure de sodium insoluble ont été filtrés et le filtrat a été mis à cristalliser deux heures au réfrigérateur. Le produit a été essoré, lavé avec 5 ml d'isopropanol glacé puis séché à l'étuve à 50° C.

Rendement = 8,6 g (79 %)
P F Kofler = 202-203° C

Le spectre RMN était semblable à celui d'un échantillon de référence.

**Revendications**

1. Nouveau procédé de préparation industrielle du N-[(1'-allyl 2'-pyrrolidinylméthyl)] 2-méthoxy 4,5-azimido benzamide caractérisé en ce que l'on traite le 2-méthoxy 4-acétylamino 5-nitro benzoate de méthyle par la soude puis par l'hydrate d'hydrazine en présence de Nickel de Raney dans le même réacteur, que l'on traite l'acide 2-méthoxy 4,5-diamino benzoïque ainsi obtenu par l'anhydride acétique pour obtenir l'acide 2-méthoxy 4,5-diacétylamino benzoïque qui est traité par le chloroformiate d'éthyle puis par la dichloropentylamine pour obtenir le N-(2',5'-dichloropentyl) 2-méthoxy 4,5-diacétylamino

benzamide que l'on fait réagir avec l'allylamine et enfin que l'on traite le N-[ (1'-allyl 2'-pyrrolidinyl)-méthyl] 2-méthoxy 4,5-diacétylamino benzamide obtenu par le nitrite de sodium.

## Claims

1. A novel process for the industrial preparation of N- [ (1'-allyl 2'-pyrrolidinylmethyl)] 2-methoxy 4,5-azimidobenzamide characterised by treating methyl 2-methoxy 4-acetylamino 5-nitro benzoate with soda and then with hydrazine hydrate in the presence of Raney nickel in the same reactor, treating the 2-methoxy 4,5-diamino benzoic acid obtained in that way with acetic anhydride to produce 2-methoxy 4,5-diacetylamino benzoic acid which is treated with ethyl chloroform ate and then with dichloropentylamine to give N-(2' ,5'-dichloropentyl) 2-methoxy 4,5-diacetylaminobenzamide which is reacted with allylamine, and finally treating the N- [ (1'-allyl 2'-pyrrolidinyl) methyl ] 2-methoxy 4,5-diacetylaminobenzamide obtained with sodium nitrite.

## Ansprüche

1. Neues Verfahren zur industriellen Herstellung von N-[(l'-Allyl-2'-pyrrolidinylmethyl)]-2-methoxy-4,5-azimido-benzamid, dadurch gekennzeichnet, daß man 2-Methoxy-4-acetylamino-5-nitro-methylbenzoat mit Natronlauge und anschließend mit Hydrazinhydrat in Gegenwart von Raney-Nickel in demselben Reaktor behandelt, daß man die so erhaltene 2-Methoxy-4,5-diamino-benzoesäure mit Acetanhydrid behandelt, um 2-Methoxy-4,5-diacetylamino-benzoesäure zu erhalten, die mit Chlorameisensäureethylester und anschließend mit Dichlorpentylamin behandelt wird, um N-(2',5'-Dichlorpentyl)-2-methoxy-4,5diacetylamino-benzamid zu erhalten, das man mit Allylamin reagieren läßt, und daß man schließlich das erhaltene N-[(1'-Allyl-2-pyrrolidinyl)methyl]-2-methoxy-4,5-diacetylaminobenzamid mit Natriumnitrit behandelt.